# EUROPEAN PATENT SPECIFICATION

(11) **EP 3 791 852 B1**
(45) Date of publication and mention of the grant of the patent: **29.10.2025**
(21) Application number: 19382793.8
(22) Date of filing: 13.09.2019
(51) Int. Cl.: A61K 8/02, A61K 8/25, A61K 8/44, A61Q 5/12

(54) **SUB-MICROPARTICLES OF SILICA CONJUGATED WITH CYSTEINE**
SUBMIKROPARTIKEL AUS MIT CYSTEIN KONJUGIERTER KIESELSÄURE
SOUS-MICROPARTICULES DE SILICE CONJUGUÉES AVEC DE LA CYSTÉINE

(43) Date of publication of application: 17.03.2021
(73) Proprietor: Infinitec Activos S.L., 08170 Montornés del Vallès, Barcelona (ES)
(72) Inventor: MOURELLE MANCINI, Marisabel, E-08028 Barcelona (ES); FOLLE, Camila, E-08015 Barcelona (ES); ALEA REYES, María Elisa, E-08025 Barcelona (ES)
(74) Representative: Evonik Patent Association

(56) References cited:
- EP-A1- 3 081 212
- CN-A- 107 603 593
- DE-A1- 102006 036 887
- US-B1- 9 408 918
- JOSHUA E. ROSEN ET AL: "Surface Functionalization of Silica Nanoparticles with Cysteine: A Low-Fouling Zwitterionic Surface", LANGMUIR, vol. 27, no. 17, 6 September 2011 (2011-09-06), pages 10507 - 10513, XP055153744, ISSN: 0743-7463, DOI: 10.1021/la201940r

## Description

### FIELD OF THE INVENTION

The present invention relates to sub-microparticles of silica conjugated with cysteine, compositions comprising said particles, their use for cosmetic applications, as well as preparation methods thereof.

### BACKGROUND OF THE INVENTION

Hair is a protein filament, made up of mainly keratin, which grows from follicles that can be found in the skin dermis. The entire human body is covered with hair, exception made to the lips, palms of the hands, soles of the feet, and some other body areas. It is a main distinguishing feature of mammals serving various physiological functions, such as thermal protection (from heat and cold), physical protection against foreign bodies (for instance eyelashes and eyebrows), and are also involved in the sense of touch providing a sense of awareness to the surroundings.

Despite all the physiological functions, in Humans hair has a very strong social function, perhaps the most relevant perceived function in human beings, being instrumental in establishing individual appearance, even playing considerable roles in social interactions, also impacting the self-esteem of an individual.

Besides physical aggression from natural sources, such as sunlight, hair can be damaged by standard and routine cosmetic procedures, such as by mechanical stress induced by combing or brushing, or hair shaping with a hair dryer or other appliances, and cosmetic hair treatments such as bleaching, dyeing and permanent waving.

Thus, it is without surprise that there is a huge availability and consistent demand for new and improved hair-care products, especially head hair care products, which are focused on restoring or maintaining the silky shine look, and smooth feel of natural healthy air.

Cysteine, characterized by its highly reactive thiol moiety, is an aminoacid widely known for its antioxidant properties. Interestingly, cysteine makes up for approximately 18% of the aminoacid content of keratin, and has found in recent times various applications in cosmetics, for instance in anti-ageing products. It has also found cosmetic uses in hair care, in permanent-wave hair treatments and hair-straightening applications. CN 107 603 593 A discloses silica nanospheres with conjugated red quantum dots that are N-acetyl-L-cysteine.The method of preparation involves mixing in water preferably at a temperature of 20-35 °C and stirring in the dark for 8-12 hours. US 9 408 918 B1 discloses mesoporous silica nanoparticles that are tethered to a cysteine.

The present invention main object is to provide sub-microparticles of silica conjugated with cysteine, and compositions comprising them for the cosmetic treatment of hair.

### SUMMARY OF THE INVENTION

The inventors have surprisingly found that compositions comprising sub-microparticles of silica conjugated with cysteine, restore smoothness to damaged hair. Surprisingly, the hair treated with the composition of the invention shows, after inducement of hair damage, a smoothness comparable to that of untreated normal healthy hair, and much higher than the one observed in hair treated with compositions comprising either cysteine, non-conjugated silica sub-microparticles or mixtures of cysteine and non-conjugated silica sub-microparticles.

The first aspect of the present invention, relates to sub-microparticles of silica conjugated with cysteine, characterized in that their mean size is between 100 and 300 nm.

In a second aspect, the present invention relates to compositions comprising the sub-microparticles according to the first aspect and water.

In a third aspect, the present invention relates to a cosmetic composition comprising the particles of the first aspect or the composition of the second aspect, and cosmetically acceptable excipients.

In a fourth aspect, the present invention relates to the use of the sub-microparticles as defined in the first aspect for the preparation of a composition according to the second aspect, or for the preparation of a cosmetic composition according to the third aspect.

In a fifth aspect, the present invention relates to the use of the composition according to the second aspect for the preparation of a cosmetic composition according to the third aspect.

In a sixth aspect, the present invention relates to the cosmetic use of the sub-microparticles according to the first aspect, the composition according to the second aspect, or the cosmetic composition according to the third aspect, for the cosmetic treatment of hair.

In a seventh aspect, the present invention relates to a procedure for the preparation of sub-microparticles according to the first aspect, comprising the steps of:
a) preparing a mixture comprising cysteine, water and silica having a mean particle size between 100 and 300 nm, preferably between 100 nm and 200 nm, more preferably between 100 nm and 150 nm and most preferably between 115 nm and 125 nm.
b) leaving the mixture prepared in a) to stand for a period of no less than 4 hours, preferably between 4 and 6 hours at a temperature comprised between 20 and 30 ° C.
c) optionally isolating the sub-microparticles conventional methods such as precipitation or centrifugation.

### DETAILED DESCRIPTION OF THE INVENTION

### Sub-microparticles of silica conjugated with cysteine

In a first aspect of the present invention, relates to sub-microparticles of silica conjugated with cysteine.

The term *"sub-microparticle"* refers to particles, wherein the mean size is comprised between 100 and 300 nm. Preferably the mean size of the sub-microparticles of the invention is comprised between 100 nm and 200 nm, more preferably between 100 nm and 150 nm, most preferably between 115 nm and 125 nm. The mean size of the particles is determined by dynamic light scattering (DLS) as explained in the example section.

The sub-microparticles of the present invention have a silica nucleus covered with cysteine. In the context of the present invention, the term *"conjugated"* refers to sub-microparticles of silica bonded to cysteine *via* an electrostatic ionic bond between the silica surface and the thiol moiety of cysteine. In an embodiment the sub-microparticles of the invention comprise an amount (in weight) of conjugated cysteine from 1% to 10%, preferably from 2% to 5% by weight of the particles.

In a preferred embodiment of the present invention the sub-microparticles are obtainable by a process comprising the steps of:
a) preparing a mixture comprising cysteine, water and silica having a mean particle size between 100 and 300 nm, preferably between 100 nm and 200 nm, more preferably between 100 nm and 150 nm and most preferably between 115 nm and 125 nm
b) leaving the mixture prepared in a) to stand for a period of no less than 4 hours, preferably between 4 and 6 hours at a temperature comprised between 20 and 30 ° C.
c) optionally isolating the sub-microparticles conventional methods such as precipitation or centrifugation.

### Intermediate compositions of the invention

In a second aspect, the present invention relates to intermediate compositions comprising the sub-microparticles according to the first aspect and water which may be used in the preparation of final cosmetic compositions.

In an embodiment, said composition is a dispersion of the functionalised sub-microparticles in water, *i.e.* a colloid. In the context of the present invention a colloid refers to a system formed by two or more phases, one fluid (liquid) and another dispersed in the form of particles, which size is comprised between 100 nm and 300 nm, thus, the particles are not observable macroscopically, but are visible on a microscopic level.

In an embodiment, the composition comprises the sub-microparticles of the invention in an amount of less than 5% by weight of the composition (w/w), preferably between 0.1% and 2.5% by weight, preferably between 0.5% and 1.5% by weight, more preferably between 0.7% and 1.0% by weight of the composition (w/w).

In another embodiment the compositions according to this aspect of the invention comprise one or more excipients selected from the group consisting of preservatives, viscosity modifiers and humectants.

In a preferred embodiment the compositions according to this aspect of the invention comprise xanthan gum, propanediol, 1,2-hexanediol and caprylyl glycol.

### Cosmetic compositions of the invention

In a third aspect, the present invention relates to a cosmetic composition comprising the particles of the first aspect or the composition of the second aspect, and cosmetically acceptable excipients.

In an embodiment, the compositions can comprise one or more cosmetic excipients selected from the group comprising surfactants, antistatic agents, moisturizing agents, antioxidants, emollients, preservatives, humectants, viscosity modifiers, perfumes, colorants, pigments, solvents and mixtures thereof. Preferred cosmetic excipients are, preservatives, viscosity modifiers, humectants, and solvents.

In the context of the present invention, a *"surfactant"* refers to a substance that diminishes the superficial tension of a composition with respect to the same composition in the absence of said component, and facilitates the uniform distribution of the composition when it is used. Surfactants suitable for the compositions of the invention may be selected from the group comprising a) non-ionic surfactants such as ethoxylated alcohols, ethoxylated fatty acids, alkyl polyglucosides, ethylene oxidepropylene oxide copolymers, fatty acid mono- or dietanolamides, fatty alcohols and amine oxides, b) amphoteric or zwitterionic surfactants such as betaines, sulfobetaines, aminopropionic acids and imidopropionic acids.

Preferred surfactants are cocamide monoisopropanolamide, cocoamidopropylbetaine, decyl glucoside, lauryl glucoside, PEG-7 glyceryl cocoate, lauryl pyrrolidone, disodium cocoamphodiacetate, sodium laureth sulphate, cetearyl alcohol, glyceryl laurate, ethoxylated oleyl-cetyl alcohol (oleth-5), polyethylene glycol diester of stearic acid (PEG-150 distearate), ethoxylated/propoxylated tridecyl ether (PPG-1 trideceth-6), alkoxylated di-ester of myristyl alcohol and adipic acid (Di-PPG-2-myreth-10 adipate) and ethoxylated hydrogenated castor oil (PEG-60 hydrogenated castor oil).

In an embodiment of the present invention, the composition may comprise antistatic agents. In the context of the present invention an *"antistatic agent"* is a compound used to reduce or eliminate the build-up of static electricity. Suitable static agents may be selected from the group comprising quaternary ammonium salts, such as dicetyldimonium chloride, and polyquaterniums (-7, -10, -11, -22, -37),

In the context of the present invention, a *"moisturizing agent"* refers to a substance which increases the water content of the skin or hair and helps to keep it soft. Examples of moisturizing agents suitable for the compositions of the invention are *Vitis vinifera* seed oil, ceramide, glucosylceramide, grape oil esters - PEG-8, glyceryl esters - cocoa butter, shea butter cetyl esters, shea butter glyceride, lauryl cocoate, and mixtures thereof.

In the context of the present invention, an *"antioxidant"* refers to a substance which inhibits or reduces reactions promoted by oxygen, thereby preventing oxidation and rancidity. Examples of antioxidants suitable for the cosmetic compositions of the invention are tocopherol, sodium tocopheryl phosphate, acetylcysteine, aloe vera plant extract, acetyl trihexylcitrate, 2-acetylhydroquinone, apo-lactoferrin and mixtures thereof. For the intermediate compositions suitable antioxidants are tocopherol and aloe vera plant extract.

In the context of the present invention, an *"emollient"* refers to a substance which softens the skin. Examples of emollients suitable for the compositions of the invention are C₁₂-C₁₅ alkyl benzoates, caprylyl glycol, propylene glycol dicaprylate/dicaprate, cetearyl alcohol, acacia dealbata flower wax, acetylarginine, acetylproline, acetylhydroxyproline, acetylated glycol stearate, algae extract, almond oil esters and propylene glycol, 1,2,6-hexanetriol, and mixtures thereof.

In the context of the present invention, a *"preservative"* refers to a substance which inhibits the development of microorganisms in the composition. Examples of preservatives suitable for the compositions of the invention are caprylyl glycol, glyceryl caprylate, glycerin, and phenylpropanol; a mixture of 1,2-hexanediol and caprylyl glycol; a mixture of phenethyl alcohol, and ethylhexylglycerin; a mixture of pentylene glycol, caprylyl glycol, and ethylhexylglycerin, and mixtures thereof.

In the context of the present invention, a *"humectant'* refers to a substance which retains humidity. Examples of humectants suitable for the compositions of the invention are D-mannitol, acetylcyclodextrin, propanediol, algae extract, 2,3-butanediol, glycerin, and propylene glycol. Preferably the humectant is propanediol.

In the context of the present invention, a *"viscosity modifier"* refers to a substance which increases the viscosity of a composition. Examples of viscosity modifiers suitable for the compositions of the invention are carbomer, carboxymethyl chitosan, propanediol, carboxymethyl dextran, steareth-30, steareth-40, steareth-50, croscarmelose, xanthan gum hydroxypropyl starch phosphate, hydroxyethyl cellulose, cocamide monoisopropanolamide (cocoamide MIPA), and mixtures thereof. Preferably the viscosity modifier is xanthan gum.

In the context of the present invention, a *"solvent"* refers to a liquid capable of dissolving or dispersing the components of the composition. Examples of solvents useful in the formulation of the compositions of the invention are 1,2-hexanediol, ethanol, isopropyl alcohol, propylene glycol, and water. Preferably the solvent is water.

In a further embodiment the cosmetic composition of the present invention comprises (a) the sub-microparticles of the invention in an amount of less than 5% by weight of the composition (w/w), preferably between 0.1% and 2.5%, preferably between 0.5% and 1.5%, more preferably between 0.7% and 1.0% by weight of the composition (w/w).

In an embodiment the cosmetic composition of the present invention further comprises at least one preservative, in an amount between 2% and 10% by weight of the composition (w/w), more preferably between 3.5% and 8.5% by weight of the composition (w/w). Preferably the at least one preservative is selected from the group comprising 1,2-hexanediol, caprylyl glycol, propanediol, and mixtures thereof.

In an embodiment, the cosmetic composition of the present invention further comprises a humectant in an amount between 2% and 5% by weight of the composition (w/w). Preferably the humectant is propanediol.

In an embodiment, the cosmetic composition of the present invention further comprises a viscosity modifier in an amount between 0.1% and 2% by weight of the composition (w/w), preferably between 0.2% and 1%, preferably between 0.3% and 0.6% by weight of the composition (w/w). Preferably the viscosity modifier is xanthan gum.

In an embodiment, the cosmetic composition of the present invention further comprises a solvent, in an amount of at least 75% by weight of the composition (w/w), preferably at least 80%, more preferably at least 85%, most preferably at least 90% by weight of the composition (w/w). Preferably the solvent is water.

In a further embodiment, the cosmetic compositions of the invention are in the form of a cream, serum, emulsion, gel, foam, paste, ointment, milk, or solution, preferably in the form of cream, solution, serum or gel.

### Procedures for the preparation of the sub-microparticles of the invention

In a further aspect, the present invention relates to a procedure for the preparation of sub-microparticles of the invention, comprising the steps of:
a) preparing a mixture comprising cysteine, water and silica having a mean particle size when measured by DLS as explained in the example section between 100 and 300 nm, preferably between 100 nm and 200 nm, more preferably between 100 nm and 150 nm and most preferably between 115 nm and 125 nm
b) leaving the mixture prepared in a) to stand for a period of no less than 4 hours, preferably between 4 and 6 hours at a temperature comprised between 20 and 30 ° C.
c) optionally isolating the sub-microparticles conventional methods such as precipitation or centrifugation.

In a particular embodiment the reaction is carried out at a temperature between 20 °C and 30 °C, more preferably between 25 °C and 30 °C.

In a particular embodiment the conjugation time is above 4 hours, more preferably between 4 and 6 hours.

The following non-limiting examples illustrate additional specific embodiments of the invention.

### EXAMPLES

### Example 1. Synthesis and characterization of sub-microparticles of silica conjugated with cysteine

### Materials and Methods

Cysteine was purchased from Sigma-Aldrich, silica sub-microparticles (20 nm, nonporous, spherical, having a bulk density of 0.08-0.10 g/cm³) were purchased from Skyspring Nanomaterials. The mean particle size of the above mentioned silica sub-microparticles when measured by DLS as explained below was 104,8 ± 9,13 nm.

In a 100 mL round bottom flask, 0.4 g of commercial silica sub-microparticles was added to 40 mL of distilled water and stirred for 5 minutes at room temperature. Then, 0.4 g of cysteine was added, and the mixture was further stirred for 4h at room temperature. Non-reacted cysteine was removed by centrifuge-washing with distilled water (5 x 10 mL), affording the conjugated sub-microparticles as a white solid. The obtained particles can be re-dispersed in a suitable amount of water to obtain a 1% (wt/v) dispersion.

The size of the sub-microparticles was determined by dynamic light scattering (DLS), using quartz cuvettes and a Zetasizer Nano ZS series (Malvern Instruments). The preparation of the samples, in case of solid samples, consists of weighing 1 mg, and suspending it in 1 mL of MilliQ H₂O. In case of liquid samples collecting a 100 µL aliquot from the sample is taken, using an automatic pipette, and added to 900 µL of MilliQ H₂O, in such a way that the sample concentration is always 1 mg/mL. Afterwards, the measurement of the particle size is performed at a temperature of 25 °C. Measurements were performed in triplicates to ensure reproducibility.

The zeta potential of the synthesized sub-microparticles was determined using DTS1070 plastic cuvettes, and a Zetasizer Nano ZS series (Malvern Instruments). The preparation of the samples, in case of solid samples, weighing 1 mg, and suspending it in 1 mL of MilliQ H₂O. In case of liquid samples consists of collecting a 100 µL aliquot from the sample is taken, using an automatic pipette, and added to 900 µL of MilliQ H₂O, in such a way that the sample concentration is always 1 mg/mL. Afterwards, the measurement of the zeta potential is performed at a temperature of 25 °C. Measurements were performed in triplicates to ensure reproducibility.

The amount of cysteine incorporation onto the conjugated sub-microparticles surface is determined by thermogravimetric analysis (TGA). The analysis is performed on a SDT Q550 TA (Waters Corporation), under an O₂ stream, a heating speed of 10 °C/min, and a heating range from 30 °C to 700 °C. Solid samples are used.

The morphology of the conjugated sub-microparticles was studied using transmission electron microscopy, using a Tecnai^{™} Spirit transmission electron microscope at 120 kV (Fei^{™} Company). Images were acquired with a Megaview III camera, and digitalized with iTEM program. The samples to be analysed were prepared by dropwise addition of the SiP-Cys water dispersion onto a carbon-covered copper rack. The samples are allowed to dry, and then the measurements are taken.

**Table I**

| Parameter | *SiP-Cys* |
|---|---|
| *Particle size (nm)* | 200-400 |
| *Mean Particle size (nm)* | 120 |
| *Zeta potential (mV)* | -12 |
| *pH* | 4-6 |
| *Cysteine incorporation (% wt*/*wt of cysteine per sub-microparticles)* | 4% |

### Example 2: Preparation of the compositions

All compositions are prepared by dispersing or dissolving the respective ingredients in distilled water, in the required amount to achieve 1% concentration (weight/volume).
**Composition A:** Particles according to example 1 - SiP-Cys.
**Composition B:** Commercial Si particles used in the preparation of example 1 (SiP).
**Composition C:** Cysteine (Cys).
**Composition D:** Commercial Si particles + Cysteine - non-conjugated (SiP, Cys).

### Example 3. Evaluation of the effect the compositions of example 2 on hair roughness

### Materials and Methods

The topography of the treated and non-treated hair samples was analysed by scanning electron microscopy (SEM). The samples to be analyzed were placed on a carbon support and subsequently covered with a thin film of amorphous carbon. Images were acquired on a JEOL JSM-7100F SEM microscope (Jeol Limited), at 5 kV with the PC-SEM version 5.1 software.

The obtained images were then processed using ImageJ image processor, which processes the 3D images obtained from the high resolution topography of the SEM. Said 3D images are then represented in pixels, which are representative of the measurement required, roughness in the present case. The image processor automatically adjusts the scale, color, etc. and removes the background of the image. The parameters are then applied to all the subsequent images to process. Non-treated hair was defined as the background, thus allowing to compare the variations of the subsequent treated hair samples. Since hair roughness is being analyzed, the pixel intensity (using the default maximum of 100% = 255 pixels) is directly correlated with said roughness (the more rough the hair surface, the higher the pixel intensity).

### Hair-bleaching protocol

30% Hydrogen Peroxide (Sigma-Aldrich) was applied to straight brown hair (NaturStyle) samples. The hair was then covered in aluminium foil, and placed on a heating plate at 40 °C, for 2 hours. After the induced damage, the bleached hair samples were washed with neutral shampoo, thoroughly rinsed with running water and subsequently air-dried.

### Treatment of damaged hair with compositions of example 2

The compositions of example 2 were then applied to the damaged hair, which was subsequently covered with aluminium foil, for 30 min at room temperature. After the treatment, the treated hair samples were washed with abundant water, air-dried, and their roughness measured according to above method.

### Results

The obtained results are shown in Table 2.

**Table 2**

| | Roughness intensity (%) | Variation *vs*. control (%) | Variation *vs*. bleached hair (%) |
|---|---|---|---|
| Control hair (non-bleached) | 35 | --- | N/A |
| Bleached Hair | 63 | +28 | --- |
| Composition **A** (SiP-Cys) | 34 | -1 | -29 |
| Composition **B** (SiP) | 56 | +21 | -7 |
| Composition **C** (Cys) | 43 | +8 | -20 |
| Composition **D** (SiP, Cys) | 45 | +10 | -18 |

| | | | |
|---|---|---|---|
| *N*/*A: Not applicable* | | | |

The results of the above table clearly show that hair treated with composition **A,** having the particles according to the present invention, is able to return to a roughness level, or in other words to a smoothness level, comparable to that of hair that has not been bleached. Moreover, composition **A** outperforms the remaining compositions, comprising its individual ingredients, together or separately, hence clearly supporting the effect observed for compositions of the invention.

### Example 4. Intermediate composition

An intermediate composition comprising the sub-microparticles of the invention was obtained by first preparing a 1% (wt/v) dispersion in distilled water, of sub-microparticles according to example 1. Then, 0.5% (wt/v) of xanthan gum (Fagron Ibérica, S.A.U), 5% (wt/v) of propanediol (Zemea^{®} propanediol, from Quimidroga, S.A), and 2% (wt/v) of a commercially available mixture of 1,2-hexanediol:caprylyl glycol (Symdiol 68^{®}, Sucesores de Jose Escuder, S.L.). A final amount of water is added to ensure a final concentration of sub-microparticles of the invention of 0.8% (wt/v).

## Claims

1. Sub-microparticles of silica conjugated with cysteine, wherein their mean size is comprised between 100 and 300 nm, wherein the mean size of the particles is determined by dynamic light scattering (DLS).

2. The sub-microparticles according to claim 1, wherein their mean size is comprised between 100 nm and 200 nm, preferably between 100 nm and 150 nm, preferably between 115 nm and 125 nm.

3. The sub-microparticles according to any one of the previous claims, wherein the amount (in weight) of conjugated cysteine is comprised from 1% to 10%, preferably from 2% to 5% by weight of the particles

4. A composition comprising the sub-microparticles as defined in any one of the previous claims and water.

5. The composition according to claim 4, comprising sub-microparticles according to any one of claims 1 to 3 in a weight percentage less than 5% by weight of the composition (w/w), preferably between 0.1% and 2.5%, preferably between 0.5% and 1.5%, more preferably between 0.7% and 1.0% by weight of the composition (w/w).

6. A cosmetic composition comprising the particles according to any one of claims 1 to 3, or the composition according to any one of claims 4 to 5 and further comprising cosmetically acceptable excipients.

7. The cosmetic composition according to claim 6, comprising sub-microparticles according to any one of claims 1 to 3 in a weight percentage less than 5% by weight of the composition (w/w), preferably between 0.1% and 2.5%, preferably between 0.5% and 1.5%, more preferably between 0.7% and 1.0% by weight of the composition (w/w).

8. The cosmetic composition according to any one of claims 6 to 7, wherein the cosmetically acceptable excipients are selected from the group comprising surfactants, antistatic agents, moisturizing agents, antioxidants, emollients, preservatives, humectants, viscosity modifiers, perfumes, colorants, pigments, and mixtures thereof.

9. The cosmetic composition according to any one of the preceding claims, comprising (a) at least one preservative, in an amount between 2% and 10% by weight of the composition (w/w); (b) a humectant in an amount between 2% and 5% by weight of the composition (w/w); (c) a viscosity modifier in an amount between 0.1% and 2% by weight of the composition (w/w); and (d) a solvent, in an amount of at least 75% by weight of the composition (w/w).

10. The cosmetic composition according to any one of the preceding claims, **characterized in that** it is in the form of a cream, a serum, an emulsion, a gel, a foam, a paste, an ointment, a milk, or a solution.

11. Use of the sub-microparticles as defined in any of claims 1 to 3 for the preparation of a composition as defined in any of claims 4 to 5.

12. Use of sub-microparticles as defined in any one of claims 1 to 3, or composition as defined in any one of claims 4 to 5, for the preparation of a cosmetic composition as defined in any one of claims 6 to 10.

13. Use of sub-microparticles as defined in any one of claims 1 to 3, or composition as defined in any one of claims 4 to 5, or cosmetic composition as defined in any one of claims 6 to 10 for the cosmetic treatment of hair.

14. Use according to claim 13, wherein the cosmetic treatment comprises reducing the roughness of hair, particularly damaged hair.

## Patentansprüche

1. Mit Cystein konjugierte Siliciumdioxid-Submikropartikel, wobei deren mittlere Größe zwischen 100 und 300 nm liegt, wobei die mittlere Größe der Partikel durch dynamische Lichtstreuung (DLS) bestimmt wird.

2. Submikropartikel nach Anspruch 1, wobei deren mittlere Größe zwischen 100 nm und 200 nm, vorzugsweise zwischen 100 nm und 150 nm, vorzugsweise zwischen 115 nm und 125 nm, liegt.

3. Submikropartikel nach einem der vorhergehenden Ansprüche, wobei die (Gewichts-)Menge an konjugiertem Cystein 1 bis 10 Gew.-%, vorzugsweise 2 bis 5 Gew.-%, der Partikel beträgt.

4. Zusammensetzung, umfassend die Submikropartikel gemäß einem der vorhergehenden Ansprüche und Wasser.

5. Zusammensetzung nach Anspruch 4, umfassend Submikropartikel nach einem der Ansprüche 1 bis 3 in einem Gewichtsprozentanteil von weniger als 5 Gew.-% der Zusammensetzung (w/w), bevorzugt zwischen 0,1 und 2,5 Gew.-%, bevorzugt zwischen 0,5 und 1,5 Gew.-%, weiter bevorzugt zwischen 0,7 und 1,0 Gew.-%, der Zusammensetzung (w/w).

6. Kosmetische Zusammensetzung, die die Partikel nach einem der Ansprüche 1 bis 3 oder die Zusammensetzung nach einem der Ansprüche 4 bis 5 umfasst und ferner kosmetisch unbedenkliche Hilfsstoffe umfasst.

7. Kosmetische Zusammensetzung nach Anspruch 6, umfassend Submikropartikel nach einem der Ansprüche 1 bis 3 in einem Gewichtsprozentanteil von weniger als 5 Gew.-% der Zusammensetzung (w/w), bevorzugt zwischen 0,1 und 2,5 Gew.-%, bevorzugt zwischen 0,5 und 1,5 Gew.-%, weiter bevorzugt zwischen 0,7 und 1,0 Gew.-%, der Zusammensetzung (w/w).

8. Kosmetische Zusammensetzung nach einem der Ansprüche 6 bis 7, wobei die kosmetisch unbedenklichen Hilfsstoffe aus der Gruppe umfassend Tenside, Antistatika, feuchtigkeitsspendende Mittel, Antioxidantien, Emollientien, Konservierungsstoffe, Feuchthaltemittel, Viskositätsmodifikatoren, Duftstoffe, Farbmittel, Pigmente und Mischungen davon ausgewählt sind.

9. Kosmetische Zusammensetzung nach einem der vorhergehenden Ansprüche, umfassend (a) mindestens einen Konservierungsstoff in einer Menge zwischen 2 und 10 Gew.-% der Zusammensetzung (w/w); (b) ein Feuchthaltemittel in einer Menge zwischen 2 und 5 Gew.-% der Zusammensetzung (w/w); (c) einen Viskositätsmodifikator in einer Menge zwischen 0,1 und 2 Gew.-% der Zusammensetzung (w/w); und (d) ein Lösungsmittel in einer Menge von mindestens 75 Gew.-% der Zusammensetzung (w/w).

10. Kosmetische Zusammensetzung nach einem der vorhergehenden Ansprüche, **dadurch gekennzeichnet, dass** sie in Form einer Creme, eines Serums, einer Emulsion, eines Gels, eines Schaums, einer Paste, einer Salbe, einer Milch oder einer Lösung vorliegt.

11. Verwendung der Submikropartikel gemäß einem der Ansprüche 1 bis 3 zur Herstellung einer Zusammensetzung gemäß einem der Ansprüche 4 bis 5.

12. Verwendung von Submikropartikeln gemäß einem der Ansprüche 1 bis 3 oder einer Zusammensetzung gemäß einem der Ansprüche 4 bis 5 zur Herstellung einer kosmetischen Zusammensetzung gemäß einem der Ansprüche 6 bis 10.

13. Verwendung von Submikropartikeln gemäß einem der Ansprüche 1 bis 3 oder einer Zusammensetzung gemäß einem der Ansprüche 4 bis 5 oder einer kosmetischen Zusammensetzung gemäß einem der Ansprüche 6 bis 10 zur kosmetischen Behandlung von Haar.

14. Verwendung nach Anspruch 13, wobei die kosmetische Behandlung die Verringerung der Rauigkeit von Haar, insbesondere geschädigtem Haar, umfasst.

## Revendications

1. Sous-microparticules de silice conjuguée à la cystéine, leur taille moyenne étant comprise entre 100 et 300 nm, la taille moyenne des particules étant déterminée par diffusion dynamique de la lumière (DLS).

2. Sous-microparticules selon la revendication 1, leur taille moyenne étant comprise entre 100 et 200 nm, de préférence entre 100 et 150 nm, et de préférence entre 115 et 125 nm.

3. Sous-microparticules selon l'une quelconque des revendications précédentes, la quantité (en poids) de cystéine conjuguée étant de 1 % à 10 %, de préférence de 2 % à 5 % en poids des particules.

4. Composition comprenant les sous-microparticules telles que définies dans l'une quelconque des revendications précédentes et de l'eau.

5. Composition selon la revendication 4, comprenant des sous-microparticules selon l'une quelconque des revendications 1 à 3, à un pourcentage en poids inférieur à 5 % en poids de la composition (p/p), de préférence entre 0,1 % et 2,5 %, de préférence entre 0,5 % et 1,5 %, plus préférentiellement entre 0,7 % et 1,0 % en poids de la composition (p/p).

6. Composition cosmétique comprenant les particules selon l'une quelconque des revendications 1 à 3, ou la composition selon l'une quelconque des revendications 4 à 5, et comprenant en outre des excipients acceptables sur le plan cosmétique.

7. Composition cosmétique selon la revendication 6, comprenant des sous-microparticules selon l'une quelconque des revendications 1 à 3, à un pourcentage en poids inférieur à 5 % en poids de la composition (p/p), de préférence entre 0,1 % et 2,5 %, de préférence entre 0,5 % et 1,5 %, plus préférentiellement entre 0,7 % et 1,0 % en poids de la composition (p/p).

8. Composition cosmétique selon l'une quelconque des revendications 6 à 7, dans laquelle les excipients acceptables sur le plan cosmétique sont choisis dans le groupe comprenant des tensioactifs, des agents antistatiques, des agents hydratants, des antioxydants, des émollients, des conservateurs, des humectants, des modificateurs de viscosité, des parfums, des colorants, des pigments et des mélanges de ceux-ci.

9. Composition cosmétique selon l'une quelconque des revendications précédentes, comprenant (a) au moins un conservateur, dans une proportion comprise entre 2 % et 10 % en poids de la composition (p/p) ; (b) un humectant dans une proportion comprise entre 2 % et 5 % en poids de la composition (p/p) ; (c) un modificateur de viscosité dans une proportion comprise entre 0,1 % et 2 % en poids de la composition (p/p) ; et (d) un solvant, dans une proportion d'au moins 75 % en poids de la composition (p/p).

10. Composition cosmétique selon l'une quelconque des revendications précédentes, **caractérisée en ce qu'**elle est sous forme de crème, de sérum, d'émulsion, de gel, de mousse, de pâte, de pommade, de lait ou de solution.

11. Utilisation des sous-microparticules telles que définies dans l'une quelconque des revendications 1 à 3 pour la préparation d'une composition telle que définie dans l'une quelconque des revendications 4 à 5.

12. Utilisation des sous-microparticules telles que définies dans l'une quelconque des revendications 1 à 3, ou de la composition telle que définie dans l'une quelconque des revendications 4 à 5, pour la préparation d'une composition cosmétique telle que définie dans l'une quelconque des revendications 6 à 10.

13. Utilisation des sous-microparticules telles que définies dans l'une quelconque des revendications 1 à 3, ou de la composition telle que définie dans l'une quelconque des revendications 4 à 5, ou de la composition cosmétique telle que définie dans l'une quelconque des revendications 6 à 10 pour le traitement cosmétique des cheveux.

14. Utilisation selon la revendication 13, le traitement cosmétique comprenant la réduction de la rugosité des cheveux, en particulier des cheveux abîmés.
